# EUROPEAN PATENT SPECIFICATION

(11) **EP 2 489 337 B1**
(45) Date of publication and mention of the grant of the patent: **06.07.2022**
(21) Application number: 12275017.7
(22) Date of filing: 17.02.2012
(51) Int. Cl.: A61F 2/915

(54) **Prosthesis and method of manufacture**
Prothese und Herstellungsverfahren dafür
Prothèse et procédé de fabrication

(30) Priority: 18.02.2011 GB 201102859
(43) Date of publication of application: 22.08.2012
(73) Proprietor: Cook Medical Technologies LLC, Bloomington, IN 47402-0489 (US)
(72) Inventor: Hansen, Palle Munk, 4632 Bjaeverskov (DK)
(74) Representative: Powell, Stephen David

(56) References cited:
- WO-A1-98/28035
- WO-A1-2006/105192
- WO-A2-03/030786
- US-A- 5 755 776

## Description

### Technical Field

The present invention relates to a generally tubular prosthesis, in particular to a prosthesis comprising a plurality of stents and to a method of manufacturing such a prosthesis.

### Background Art

Prostheses have been used for many years to treat a number of vascular medical conditions. Prostheses in common use comprise at least one stent and are either of the self-expandable type, made for example of a shape memory material, or separately expandable, such as by balloon expansion.

Such prostheses are designed to fit within the vasculature of the patient and need to be appropriate for the size and shape of the lumen. For this purpose, they must be conformable and must not be of a nature that they can apply against the vessel walls forces which could damage or adversely affect the functionality of the vessel or of other organs nearby.

Preferably, such prostheses have a small cross-sectional diameter and/or profile for introducing the prosthesis into the affected body lumen. A configuration which is extremely suited for implantation in a body lumen is a generally cylindrical prosthesis which can radially expand from a first, small, collapsed diameter to a second, larger, expanded diameter. Such prostheses can be deployed in a body lumen at the desired location by means of an introducer assembly, whereby the prosthesis is placed on a catheter and transported through the lumen to the desired location. The prosthesis is retained in a compressed condition by a sheath. Once the introducer assembly has been transported to the desired deployment position the sheath is withdrawn, thus releasing the prosthesis so that

it can expand radially into an expanded condition. In this regard, the prosthesis may be self-expanding or the catheter may be provided with a balloon or another expansion mechanism which exerts a radial outward pressure on the prosthesis so that the prosthesis expands to a larger diameter.

Lateral deflection in such prostheses can occur during deployment, such as when the prosthesis is longitudinally compressed as it is maneuvered into the desired location in a patient's lumen. Such lateral deflection can be problematic during deployment of the prosthesis. For example, this can distort the sheath to cause increased friction between the sheath and the lumen wall, thus hindering deployment. This may particularly be the case when the prosthesis is to be deployed in a curved lumen, such as the aortic arch, and in extreme cases lateral deflection can prevent the prosthesis from being located in the desired position. Further, such lateral deflection can be dangerous for the patient in more delicate applications, such as in smaller and more delicate vessels including, for instance, cerebral vessels.

Prostheses with various stent arrangements are disclosed in US 7637935, US 2009/0204201, WO 98/20810, US 5776181, US 2005/0149168, US 2007/0244543, US 7534258 and US 6656220.

WO 2006/105192, WO 03/030786, US 5755776 and WO 98/28035 disclose prostheses with stents having shaped apex portions.

### Disclosure of The Invention

Aspects of the present invention seek to provide an improved prosthesis in which the aforementioned problems associated with lateral deflection are substantially reduced or overcome.

According to a first aspect of the present invention, there is provided a substantially tubular prosthesis as in claim 1.

The shaped portion of the apexes advantageously prevents interdigitation of adjacent stent members when the prosthesis is subjected to longitudinal compression forces.

In a preferred embodiment the shaped portions are substantially planar, and may be substantially perpendicular to the longitudinal direction of the prosthesis.

The proximal apexes of the first stent member may be substantially parallel to the distal apexes of the second stent member.

The strut elements may be substantially straight, and may be substantially parallel when the prosthesis is in a compressed configuration.

In an embodiment, each stent member is spaced apart from an adjacent stent member by at least one connector.

Alternatively, or in addition, the stent members may be fastened to a graft material to form a stent graft. The stent members may be fastened to the graft such that each stent member is spaced apart from an adjacent stent member.

When the stent members are spaced apart from an adjacent stent member by at least one connector, the prosthesis advantageously further comprises at least one engagement member located at an end of the prosthesis, the engagement member comprising a lateral extension which extends adjacent the distal apexes of the first stent member such that the lateral extension engages the distal apexes of the first stent member when the prosthesis is subjected to longitudinal compression forces, the lateral extension being shaped to form a substantially planar engagement surface facing the distal apexes of the first stent member.

This arrangement maximises the potential contact area between the engagement member and the distal apexes of the first stent member, and causes the engagement member to engage the distal apexes of the first stent member. The distal apexes are prevented from pushing past the lateral extension, and this arrangement allows a portion of the longitudinal compression force to be distributed to parts of the prosthesis that are not directly connected to the connector.

According to a second aspect of the present invention, there is provided a method of manufacturing a substantially tubular prosthesis as in claim 10.

The shaped portions may be substantially planar.

The proximal apexes of the first stent member may be arranged to engage the distal apexes of the adjacent second stent member when the longitudinal compression forces are of a predetermined magnitude.

The prosthesis may be formed into a stent graft in which the stent members are fastened to a graft material.

The connectors may be arranged such that the proximal apexes of the first stent member are arranged to engage the distal apexes of the adjacent second stent member when the longitudinal compression forces are of a predetermined magnitude.

### Brief Description of the Drawings

Preferred embodiments of the invention will now be described, by way of example only, with reference to the accompanying drawings of which:
Figure 1 shows a developed view of a portion of a prior art prosthesis;
Figure 2 shows a detailed portion of the prosthesis of Figure 1;
Figure 3 shows detail of the prosthesis of Figure 1 in schematic form, when the prosthesis is experiencing longitudinal compression;
Figure 4 shows an arrangement of a portion of a prosthesis in accordance with a first embodiment of the present invention;
Figure 5 shows a detailed portion of the prosthesis of Figure 4;
Figures 6 to 8 show modified apex portions of prostheses in accordance with the present invention;
Figure 9a shows a portion of a prosthesis in accordance with a second embodiment of the present invention; and
Figure 9b shows a modification of the prosthesis of Figure 9a.

### Description of the Preferred Embodiments

For the purposes of this disclosure, the term "prosthesis" means any replacement for a body part or function of that body part. It can also be used to refer to a device that enhances or adds functionality to a physiological system.

The term "stent" or "stent member" means any device or structure that adds rigidity, expansion force or support to a prosthesis or body lumen.

The term "stent graft" refers to a prosthesis comprising a graft material that forms a lumen through at least a portion of its length and has a number of stent members attached thereto.

Further, when used in connection with description of a stent, stent graft or other implantable medical device or prosthesis, the term "proximal" refers to a part or position closest to the patient's heart, that is upstream in the direction of blood flow, when the prosthesis is in situ, while the term "distal" refers to a part or position furthest from the heart.

In general, prostheses according to the present invention may comprise a plurality of longitudinally spaced apart stents. Each stent may be formed from a resilient wire such as Nitinol wire and may be of generally zig-zag shape, comprising a plurality of struts with a bend between each pair of struts.

Nitinol is a shape memory metal formed from a nickel-titanium (NiTi) alloy that "remembers" its geometry. The wire may be formed into the desired zig-zag shape and then heat treated to retain that shape. After cooling, if it is deformed, it will return to the desired zig-zag shape. Thus, stents formed from such material are able to be radially compressed, for example so as to allow their deployment, and will return to a desired expanded form once the compression forces have been removed.

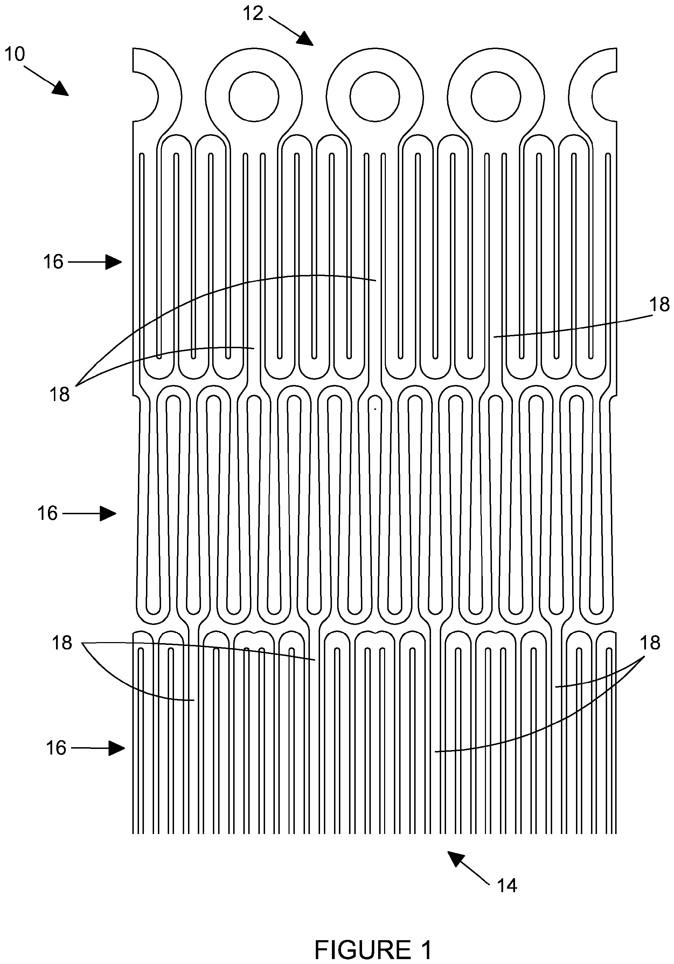

Referring now to Figure 1 of the accompanying drawings, a portion of a prior art prosthesis 10 is shown. The prosthesis 10 comprises a distal end 12 and a proximal end 14, with a plurality of stent members or stents 16 spaced apart along the length of the prosthesis 10. Each stent 16 is spaced apart from the adjacent stent(s) 16 in the longitudinal direction of the prosthesis 10 by connectors 18. The connectors 18 are flexible enough to allow for curvature of the prosthesis 10 during longitudinal compression such as that experienced during deployment.

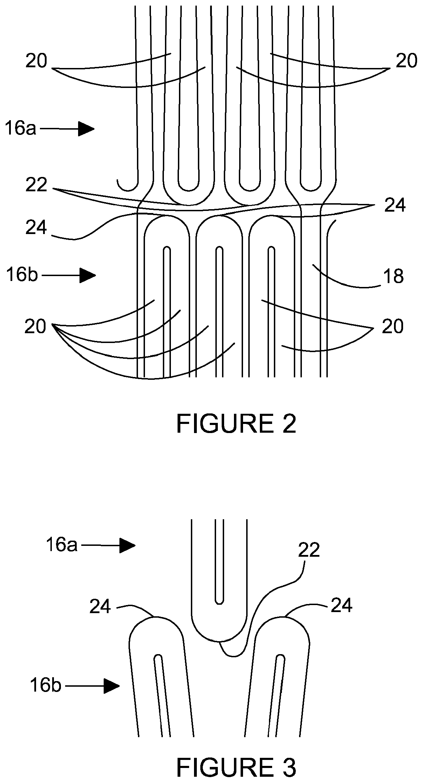

As can be seen from Figure 2, each stent 16 is formed from a plurality of struts 20 with a bend between each pair of struts 20. The bends form apexes, and the stents 16 are arranged such that the proximal apexes 22 of one stent 16a are facing the distal apexes 24 of the adjacent stent 16b. Adjacent stents 16a, 16b are in a staggered arrangement in which the proximal apexes 22 of one stent 16a are laterally offset from the distal apexes 24 of the adjacent stent 16b. The apexes are of generally rounded form.

Only two stents 16a and 16b are shown in Figure 2, although this is for clarity purposes only and it will be understood that the prosthesis 10 may in reality comprise more stents 16 spaced apart along the length of the prosthesis 10.

The prior art prosthesis 10 shown in Figure 2 is in the compressed configuration. The prosthesis 10 will be in this configuration during its deployment in a lumen, and will in practice be held in the compressed configuration by means of a sheath (not shown) which forms a part of the introducer assembly.

Once the introducer assembly is in the desired deployment position, the sheath is withdrawn, thus releasing the prosthesis 10 so that it can expand radially into an expanded condition. The sheath can then be withdrawn completely from the patient.

During deployment of the prosthesis 10, the compressed prosthesis 10 will be pushed longitudinally through the patient's vasculature by the introducer assembly. The prosthesis 10 will be subjected to longitudinal compression forces, and will thus experience longitudinal compression. When the prosthesis 10 is subjected to such longitudinal compression forces, the proximal apexes 22 of the stent 16a move towards the distal apexes 24 of the adjacent stent 16b such that the space between adjacent stents diminishes.

There are also situations in which the prosthesis may be subjected to longitudinal compression forces other than during deployment. One such situation is when the prosthesis is under development and is being tested by a design engineer in a laboratory. Another situation in which the prosthesis may be subjected to longitudinal compression forces is when the prosthesis is being tested after manufacture, to establish whether or not the manufactured prosthesis is fit for purpose prior to this prosthesis being made available for installation in a patent.

The connectors 18 must be flexible enough to allow for curvature of the prosthesis 10 during deployment. As such, the connectors are not stiff enough to prevent engagement of adjacent stents 16a, 16b.

Continued longitudinal compression of the prosthesis 10, to the magnitude of that experienced during deployment (whether this compression results from deployment of the prosthesis or not), causes adjacent stents 16a, 16b to firstly engage and then to interdigitate or wedge. That is to say, the proximal apexes 22 of one stent 16a will push past the distal apexes 24 of the adjacent stent 16b such that the struts 20 lie next to each other in the radial direction and the stents 16a, 16b overlap. The rounded form of the proximal 22 and distal 24 apexes allows the apexes to push past each other and interdigitate.

Figure 3 shows, schematically, a portion of the prosthesis 10 in the compressed configuration, under the influence of longitudinal compression forces. The stents 16a, 16b are beginning to interdigitate as described above. Such interdigitation causes lateral deflection of the prosthesis 10. It will be understood that this lateral deflection will be magnified if interdigitation occurs throughout the prosthesis 10.

Such lateral deflection of the prosthesis 10 during deployment thereof is undesirable, as it can cause the prosthesis 10 to press upon the inner surface of the sheath to cause increased friction between the outer surface of the sheath and the lumen wall, thus hindering deployment. This may particularly be the case when the prosthesis 10 is to be deployed in a curved lumen, such as the aortic arch, and in extreme cases lateral deflection can prevent the stent from being located in the desired position. Further, such lateral deflection can be dangerous for the patient in more delicate applications, such as in smaller and more delicate vessels including, for instance, cerebral vessels.

This lateral deflection may be compounded by the action of the connectors 18 when subjected to longitudinal compression. In this regard, the connectors 18 deform under longitudinal compression and this deformation could cause at least a portion of the connectors 18 to press upon the inner surface of the sheath to further increase the friction between the outer surface of the sheath and the lumen wall, thus further hindering deployment.

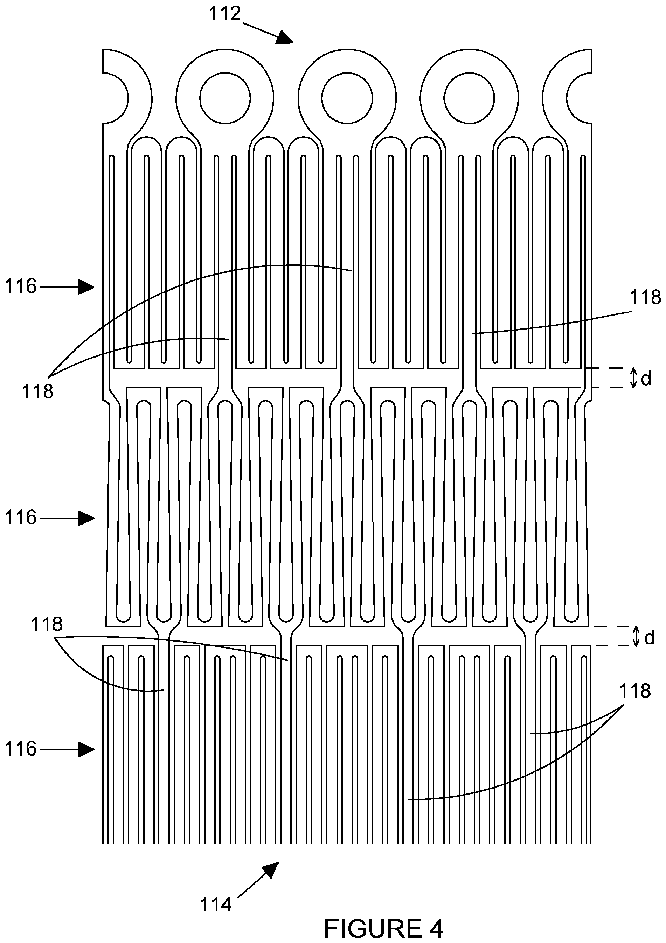

Figure 4 shows a portion of a prosthesis 110 according to an embodiment of the present invention. As with the prior art prosthesis shown in Figures 1 to 3, the prosthesis 110 comprises a distal end 112 and a proximal end 114, with a plurality of stents 116 spaced apart along the length of the prosthesis 110.

Each stent 116 is spaced apart from the adjacent stent(s) 116 in the longitudinal direction of the prosthesis 110 by connectors 118. The connectors 118 are flexible enough to allow for curvature of the prosthesis 110 during deployment. Each stent 116 is spaced apart from the adjacent stent(s) 116 in the longitudinal direction of the prosthesis 110 by a distance "d". In this embodiment, "d" is 0.1 mm. The longitudinal length of the stents 116 in this embodiment is 2 mm. As such, the distance "d" is 5% of the longitudinal length of the stents 116.

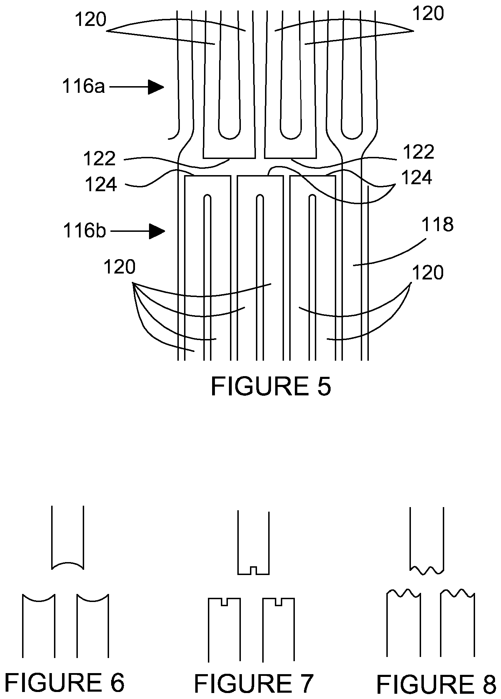

As can be seen from Figure 5, each stent 116 is formed into a generally zig-zag shape, and comprises a plurality of struts 120 with an apex 122, 124 between each pair of struts 120. The struts 120 are substantially straight. The apexes 122, 124 have a generally flat or flattened shape, that is to say that, instead of forming a generally rounded bend, the apexes between pairs of struts 120 form a more flattened, planar shape. In the present embodiment, the connection between pairs of struts 120 is substantially straight. The stents 116 are arranged such that the proximal apexes 122 of one stent 116a are facing the distal apexes 124 of the adjacent stent 116b, and the shaped or flat portions of the proximal apexes 122 and distal apexes 124 are substantially parallel. Further, the shaped or flat portions of the proximal apexes 122 and distal apexes 124 are substantially perpendicular to the longitudinal direction of the prosthesis 110.

According to the invention, adjacent stents 116a, 116b are in a staggered arrangement in which the proximal apexes 122 of one stent 116a are laterally offset from the distal apexes 124 of the adjacent stent 116b.

Figure 5 shows a detailed portion of the prosthesis 110 in the compressed configuration. When in this compressed configuration, each strut 120 lies substantially parallel in side-by-side relation to each other strut 120 of the same stent 116. The prosthesis 110 is in the compressed configuration during its deployment in a lumen, and is in practice held in the compressed configuration by means of a sheath (not shown) which forms a part of the introducer assembly.

During deployment, the compressed prosthesis 110 is pushed longitudinally through the patient's vasculature by the introducer assembly. The prosthesis 110 is subjected to longitudinal compression forces, and thus experiences longitudinal compression. The flat shape of the proximal 122 and distal 124 apexes provides respective abutment surfaces for adjacent stents 116a, 116b, such that when the prosthesis 110 is subjected to such longitudinal compression forces the flat and parallel surfaces of the proximal apexes 122 of the stent 116a engage and abut the flat and parallel surfaces of the distal apexes 124 of the adjacent stent 116b.

There are also situations in which the prosthesis may be subjected to longitudinal compression forces other than during deployment. Examples of such situation are during development of the prosthesis and during testing of the prosthesis after manufacture, as described hereinbefore.

Engagement of the shaped or flat proximal apexes 122 of one stent 116a with the shaped or flat distal apexes 124 of the adjacent stent 116b also acts to prevent excessive deformation of the connectors 118.

An advantage of the above described arrangement is therefore that the proximal apexes 122 of one stent 116a are prevented from pushing past the distal apexes 124 of the adjacent stent 116b. Thus, interdigitation of the struts 120 is avoided. The avoidance of interdigitation in this way leads in turn to a prevention of lateral deflection of the prosthesis 110 during longitudinal compression, for example during deployment of the prosthesis 110. The integrity of the prosthesis 110 shape is therefore maintained during deployment.

Another advantage of the above described arrangement is that excessive deformation of the connectors 118 is prevented. Deformation of the connectors could in itself lead to lateral deflection of the prosthesis 110.

A further advantage of the above described arrangement is that the effect of the prosthesis 110 upon the sheath during deployment is substantially reduced or eliminated. As a result, friction between the sheath and the lumen wall, which can be problematic and/or dangerous during deployment of the prosthesis 110, is substantially reduced or eliminated.

In addition, it is believed that the arrangement described herein will lead to a decrease in the force required to deploy the prosthesis. It is therefore believed that the present invention will find application in drug eluting stents, such as the present applicant's Zilver PTX stents, which are known to cause a significant increase in the force necessary for deployment of the stent. That is to say, the increased force necessary for deployment of a drug eluting stent may be at least partially negated by arranging the stent in accordance with the present invention.

In a modified arrangement, the stents may be formed from any suitable shape memory alloy. Alternatively, the stents may be formed from a metal such as stainless steel, or a suitable plastics material.

In a further modified arrangement, the stents may be stitched or otherwise fastened onto a tubular graft material, thus forming a stent graft. In this arrangement, connectors are not required to position the stents longitudinally along the prosthesis.

Further, although in the preferred embodiment the stents are spaced apart by a distance "d" of 0.1 mm, in modified arrangements the spacing "d" may be at least zero and may be 0.2 mm or less, and is preferably 0.1 mm or less. As such, the stents could be in contact even when the prosthesis is not subjected to longitudinal compression forces.

In addition, the longitudinal length of the stents is 2 mm in the preferred embodiment, however the stent length may be at least 1 mm, preferably at least 1.4 mm, and may be 3 mm or less, preferably 2.5 mm or less.

Although the shaped apexes in the preferred embodiment are flat, in modifications the shaped apexes could take on another form. The shaped portion may comprise the whole of, or a part of, the apex. The apexes are shaped such that the apexes of one stent engage or abut the apexes of an adjacent stent, and are thus prevented from pushing past the apexes of the adjacent stent, when the prosthesis is subjected to longitudinal compression forces. Examples of alternative forms of shaped apexes are concave (see Figure 6), castellated (see Figure 7) and undulating (see Figure 8). In these alternate forms, at least a portion of the apex is flat or concave.

According to the invention, the stents are spaced apart from the neighbouring stent(s) by a spacing "d" which is of the order of between zero and 20% of the longitudinal length of the stents. It will be appreciated that a zero spacing corresponds to the apexes being in contact with each other even before longitudinal compression occurs.

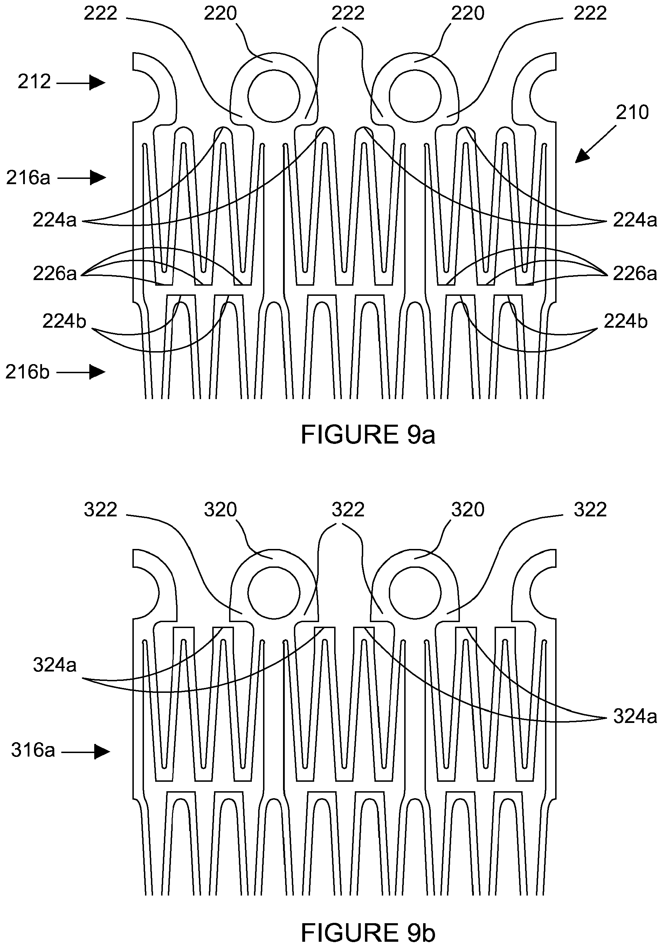

Figure 9a shows a portion of a prosthesis 210 according to a second embodiment of the present invention. The prosthesis 210 comprises a distal end 212 and a proximal end, with a plurality of stents 216a, 216b spaced apart along the length of the prosthesis 210. In this embodiment, markers 220 are provided at the proximal and/or distal ends of the prosthesis 210 to assist the physician in positioning the prosthesis during a medical procedure. These markers 220 are also used as engagement members, so as to provide a contact surface at the end(s) of the prosthesis 210 that may be pushed against, for example when the prosthesis 210 is longitudinally compressed, so that the prosthesis 210 structure itself is not directly pushed against. The markers/engagement members 220 shown in Figure 9a include lateral extensions 222 which extend from the body of the engagement members 220 such that the lateral extensions 222 are located adjacent the distal apexes 224a of the stent 216a (that is, the apexes which are located at the distal end 212 of the prosthesis 210). These lateral extensions 222 are provided so as to increase the potential contact area between the engagement members 220 and the apexes 224a, and to cause the engagement members 220 to engage the apexes 224a, thus preventing the apexes 224a from pushing past the lateral extensions 222 of the engagement members 220.

In addition, the proximal apexes 226a of the stent 216a and the distal apexes 224b of the stent 216b have a generally flat or flattened shape, that is to say that, instead of forming a generally rounded bend, the apexes between pairs of struts form a more flattened, planar shape. The stents 216a, 216b are arranged such that the proximal apexes 226a of one stent 216a face the distal apexes 224b of the adjacent stent 216b, and the shaped or flat portions of the proximal apexes 226a and distal apexes 224b are substantially parallel. Further, the shaped or flat portions of the proximal apexes 226a and distal apexes 224b are substantially perpendicular to the longitudinal direction of the prosthesis 210.

Figure 9b shows a modification of the embodiment shown in Figure 9a. Only those elements which have been modified have been given reference numerals in Figure 9b; all other elements are similar to those described in connection with Figure 9a. In this modification, the engagement members 320 are modified in that the surfaces of the lateral extensions 322 which face the apexes 324a are planar. The distal apexes 324a of the stent 316a are also modified so as to have a generally flat or flattened shape, such that the distal apexes 324a of the stent 316a are similar to the proximal apexes 226a and distal apexes 224b shown in Figure 9a. This modification further increases the likelihood of engagement between the planar lateral extensions 322 of the engagement member 320 and the flattened distal apexes 324a of the stent 316a when the prosthesis is subjected to longitudinal compression forces, thus preventing the distal apexes 324a from pushing past the lateral extensions 322.

With prior art arrangements such as that shown in Figure 1, longitudinal compression force applied to the prosthesis via the engagement member(s) would be concentrated only on the connector associated with a particular engagement member and the struts adjacent thereto, that is those structural members which are directly connected to an engagement member. Such a concentration of force can lead to bending and deformation of the connector and struts as the prosthesis is longitudinally compressed, for example as it is pushed into a delivery system or released from the delivery system for implantation.

The arrangements shown in Figures 9a and 9b cause the longitudinal compression force to be distributed to struts which are not directly connected to the engagement member in addition to those connectors and struts which are directly connected to an engagement member. The longitudinal compression force is thus directed to more of the structural members of the prosthesis, and thus laterally across the prosthesis. In addition, due to the flat shape of the stent apexes, longitudinally adjacent stent members are caused to engage upon the application of a longitudinal compression force. Such engagement causes the applied force to be distributed longitudinally throughout the prosthesis as well as being spread out laterally. The combination of a shaped engagement member and shaped apexes can therefore be seen to work synergistically to distribute the compression force both laterally and longitudinally throughout the prosthesis.

Because the longitudinal compression force is spread out more evenly across the prosthesis, bending and deformation of the connectors and struts of the prosthesis is minimised. This is especially advantageous when prostheses of longer length are used, since such prostheses typically require the application of greater pushing forces due to increased friction.

## Claims

1. A substantially tubular prosthesis (110; 210) comprising a plurality of stent members (116, 116a, 116b; 216a, 216b; 316a) positioned longitudinally along the prosthesis, each stent member comprising a plurality of strut elements (120) connected by apexes (122, 124; 224a, 224b, 226a; 324a), the prosthesis being arranged such that proximal apexes (122; 226a) of a first stent member (116a; 216a) engage distal apexes (124; 224b) of a longitudinally adjacent second stent member (116b; 216b) when the prosthesis is subjected to longitudinal compression forces, each of the proximal apexes of the first stent member and the distal apexes of the second stent member having a shaped portion which is flat or concave and having a width, so as to present a line of engagement surfaces to the distal apexes of the second stent member and the proximal apexes of the first stent member respectively, wherein gaps between circumferentially adjacent engagement surfaces are narrower than the widths of the shaped portions when the prosthesis is in a compressed configuration;
wherein each of the plurality of stent members is spaced apart from an adjacent stent member by a spacing (d) of the order of between zero and 20% of a longitudinal length of the stent members in a compressed configuration of the prosthesis;
wherein the adjacent stent members are in a staggered arrangement in which proximal apexes of one stent member are laterally offset from distal apexes of the adjacent stent member.

2. A prosthesis according to claim 1, wherein the shaped portions are substantially planar.

3. A prosthesis according to claim 2, wherein the shaped portions are substantially perpendicular to the longitudinal direction of the prosthesis (110; 210).

4. A prosthesis according to claim 2 or 3, wherein the proximal apexes (122; 226a) of the first stent member (116a; 216a) are substantially parallel to the distal apexes (124; 224b) of the second stent member (116b; 216b).

5. A prosthesis according to any preceding claim, wherein the strut elements (120) are substantially parallel when the prosthesis is in a compressed configuration.

6. A prosthesis according to any preceding claim, wherein each stent member (116, 116a, 116b; 216a, 216b; 316a) is spaced apart from an adjacent stent member by at least one connector (118).

7. A prosthesis according to claim 6, further comprising at least one engagement member (220; 320) located at an end of the prosthesis, the engagement member comprising a lateral extension (222; 322) which extends adjacent the distal apexes (224a; 324a) of the first stent member (216a; 316a) such that the lateral extension engages the distal apexes of the first stent member when the prosthesis is subjected to longitudinal compression forces, the lateral extension being shaped to form a substantially planar engagement surface facing the distal apexes of the first stent member.

8. A prosthesis according to any preceding claim, wherein the stent members (116, 116a, 116b; 216a, 216b; 316a) are fastened to a graft material to form a stent graft.

9. A prosthesis according to claim 8, wherein the stent members (116, 116a, 116b; 216a, 216b; 316a) are fastened to the graft such that each stent member is spaced apart from an adjacent stent member.

10. A method of manufacturing a substantially tubular prosthesis (110; 210), the method comprising the steps of:
forming a plurality of stent members (116, 116a, 116b; 216a, 216b; 316a) positioned longitudinally along the prosthesis, wherein each stent member comprises a plurality of strut elements (120) connected by apexes (122, 124; 224a;, 224b, 226a; 324a);
arranging the prosthesis such that proximal apexes (122; 226a) of a first stent member (116a; 216a) engage distal apexes (124; 224b) of a longitudinally adjacent second stent member (116b; 216b) when the prosthesis is subjected to longitudinal compression forces; and
forming each of the proximal apexes of the first stent member and the distal apexes of the second stent member to have a shaped portion which is flat or concave and to have a width, so as to present a line of engagement surfaces to the distal apexes of the second stent member and the proximal apexes of the first stent member respectively, wherein gaps between circumferentially adjacent engagement surfaces are narrower than the widths of the shaped portions when the prosthesis is in a compressed configuration;
wherein each of the plurality of stent members is spaced apart from an adjacent stent member by a spacing (d) of the order of between zero and 20% of a longitudinal length of the stent members in a compressed configuration of the prosthesis;
wherein the adjacent stent members are in a staggered arrangement in which proximal apexes of one stent member are laterally offset from distal apexes of the adjacent stent member.

11. A method according to claim 10, wherein the shaped portions are formed to be substantially planar.

12. A method according to claim 11, wherein the shaped portions are formed to be substantially perpendicular to the longitudinal direction of the prosthesis (110; 210).

13. A method according to claim 11 or 12, wherein the proximal apexes (122; 226a) of the first stent member (116a; 216a) are formed to be substantially parallel to the distal apexes (124; 224b) of the second stent member (116b; 216b).

14. A method according to any one of claims 10 to 13, wherein the stent members (116, 116a, 116b; 216a, 216b; 316a) are formed such that the strut elements (120) are substantially parallel when the prosthesis (110; 210) is in a compressed configuration.

15. A method according to any one of claims 10 to 14, wherein the prosthesis (110; 210) is formed such that each stent member (116, 116a, 116b; 216a, 216b; 316a) is spaced apart from an adjacent stent member by at least one connector (118).

## Patentansprüche

1. Im Wesentlichen rohrförmige Prothese (110; 210), umfassend eine Vielzahl von Stentgliedern (116, 116a, 116b; 216a, 216b; 316a), die in Längsrichtung entlang der Prothese positioniert sind, wobei jedes Stentglied eine Vielzahl von Strebenelementen (120) umfasst, die über Scheitel (122, 124; 224a, 224b, 226a, 324a) verbunden sind, wobei die Prothese so angeordnet ist, dass die proximalen Scheitel (122; 226a) eines ersten Stentglieds (116a; 216a) distale Scheitel (124; 224b) eines in Längsrichtung benachbarten zweiten Stentglieds (116; 216b) in Eingriff nehmen, wenn die Prothese Längskompressionskräften ausgesetzt wird, wobei jeder der proximalen Scheitel des ersten Stentglieds und der distalen Scheitel des zweiten Stentglieds einen geformten Abschnitt haben, der flach oder konkav ist, und eine Breite haben, um eine Linie von Eingriffsflächen zu den distalen Scheiteln des zweiten Stentglieds bzw. den proximalen Scheiteln des ersten Stentglieds aufzuweisen, wobei Spalte zwischen in Umfangsrichtung benachbarten Eingriffsflächen schmaler als die Breiten der geformten Abschnitte sind, wenn die Prothese in einer komprimierten Konfiguration ist,
wobei jedes der Vielzahl von Stentgliedern von einem benachbarten Stentglied mit einem Abstand (d) in einer Größenordnung zwischen 0 und 20% einer in Längsrichtung gehenden Länge der Stentglieder in einer komprimierten Konfiguration der Prothese beabstandet ist,
wobei die benachbarten Stentglieder in einer gestuften Anordnung sind, in der proximale Scheitel eines Stentglieds seitlich von distalen Scheiteln des benachbarten Stentglieds versetzt sind.

2. Prothese nach Anspruch 1, wobei die geformten Abschnitte im Wesentlichen planar sind.

3. Prothese nach Anspruch 2, wobei die geformten Abschnitte im Wesentlichen senkrecht zu der Längsrichtung der Prothese (110; 210) verlaufen.

4. Prothese nach Anspruch 2 oder 3, wobei die proximalen Scheitel (122; 226a) des ersten Stentglieds (116a; 216a) im Wesentlichen parallel zu den distalen Scheiteln (124; 224b) des zweiten Stentglieds (116b; 216b) verlaufen.

5. Prothese nach einem der vorhergehenden Ansprüche, wobei die Strebenelemente (120) im Wesentlichen parallel verlaufen, wenn die Prothese in einer komprimierten Konfiguration ist.

6. Prothese nach einem der vorhergehenden Ansprüche, wobei jedes Stentglied (116, 116a, 116b; 216a, 216b; 316a) von einem benachbarten Stentglied durch mindestens einen Verbinder (118) beabstandet ist.

7. Prothese nach Anspruch 6, ferner umfassend mindestens ein Eingriffsglied (220; 320), das an einem Ende der Prothese angeordnet ist, wobei das Eingriffsglied eine seitliche Verlängerung (222; 322) umfasst, die sich den distalen Scheiteln (224a; 324a) des ersten Stentglieds (216a; 316a) benachbart erstreckt, so dass die seitliche Verlängerung die distalen Scheitel des ersten Stentglieds in Eingriff nimmt, wenn die Prothese Längskompressionskräften ausgesetzt wird, wobei die seitliche Verlängerung so gestaltet ist, dass sie eine im Wesentlichen planare Eingriffsfläche bildet, die den distalen Scheiteln des ersten Stentglieds zugewandt ist.

8. Prothese nach einem der vorhergehenden Ansprüche, wobei die Stentglieder (116, 116a, 116b; 216a, 216b; 316a) zur Bildung eines Stentgrafts an einem Graftmaterial befestigt sind.

9. Prothese nach Anspruch 8, wobei die Stentglieder (116, 116a, 116b; 216a, 216b; 316a) an dem Graft befestigt sind, so dass jedes Stentglied von einem benachbarten Stentglied beabstandet ist.

10. Verfahren zur Herstellung einer im Wesentlichen rohrförmige Prothese (110; 210), wobei das Verfahren die folgenden Schritte umfasst:
Bilden einer Vielzahl von Stentgliedern (116, 116a, 116b; 216a, 216b; 316a), die in Längsrichtung entlang der Prothese positioniert sind, wobei jedes Stentglied eine Vielzahl von Strebenelementen (120) umfasst, die über Scheitel (122, 124; 224a, 224b, 226a, 324a) verbunden sind,
Anordnen der Prothese, so dass die proximalen Scheitel (122; 226a) eines ersten Stentglieds (116a; 216a) distale Scheitel (124; 224b) eines in Längsrichtung benachbarten zweiten Stentglieds (116; 216b) in Eingriff nehmen, wenn die Prothese Längskompressionskräften ausgesetzt wird, und
Formen jedes der proximalen Scheitel des ersten Stentglieds und der distalen Scheitel des zweiten Stentglieds, so dass sie einen geformten Abschnitt haben, der flach oder konkav ist, und eine Breite haben, um eine Linie von Eingriffsflächen zu den distalen Scheiteln des zweiten Stentglieds bzw. den proximalen Scheiteln des ersten Stentglieds aufzuweisen, wobei Spalte zwischen in Umfangsrichtung benachbarten Eingriffsflächen schmaler als die Breiten der geformten Abschnitte sind, wenn die Prothese in einer komprimierten Konfiguration ist,
wobei jedes der Vielzahl von Stentgliedern von einem benachbarten Stentglied mit einem Abstand (d) in einer Größenordnung zwischen 0 und 20% einer in Längsrichtung gehenden Länge der Stentglieder in einer komprimierten Konfiguration der Prothese beabstandet ist,
wobei die benachbarten Stentglieder in einer gestuften Anordnung sind, in der proximale Scheitel eines Stentglieds seitlich von distalen Scheiteln des benachbarten Stentglieds versetzt sind.

11. Verfahren nach Anspruch 10, wobei die geformten Abschnitte im Wesentlichen planar ausgebildet werden.

12. Verfahren nach Anspruch 11, wobei die geformten Abschnitte im Wesentlichen senkrecht zu der Längsrichtung der Prothese (110; 210) verlaufend ausgebildet werden.

13. Verfahren nach Anspruch 11 oder 12, wobei die proximalen Scheitel (122; 226a) des ersten Stentglieds (116a; 216a) im Wesentlichen parallel zu den distalen Scheiteln (124; 224b) des zweiten Stentglieds (116b; 216b) verlaufend ausgebildet sind.

14. Verfahren nach einem der Ansprüche 10 bis 13, wobei die Stentglieder (116, 116a, 116b; 216a, 216b; 316a) so ausgebildet werden, dass die Strebenelemente (120) im Wesentlichen parallel verlaufen, wenn die Prothese (110; 210) in einer komprimierten Konfiguration ist.

15. Verfahren nach nach einem der Ansprüche 10 bis 14, wobei die Prothese (110; 210) so ausgebildet wird, dass jedes Stentglied (116, 116a, 116b; 216a, 216b; 316a) von einem benachbarten Stentglied durch mindestens einen Verbinder (118) beabstandet ist.

## Revendications

1. Prothèse sensiblement tubulaire (110 ; 210) comprenant une pluralité d'éléments de stent (116, 116a, 116b ; 216a, 216b ; 316a) positionnés longitudinalement le long de la prothèse, chaque élément de stent comprenant une pluralité d'éléments d'entretoise (120) reliés par des sommets (122, 124 ; 224a, 224b, 226a ; 324a), la prothèse étant agencée de telle sorte que les sommets proximaux (122 ; 226a) d'un premier élément de stent (116a ; 216a) viennent en prise avec des sommets distaux (124 ; 224b) d'un second élément de stent (116b ; 216b) adjacent longitudinalement lorsque la prothèse est soumise à des forces de compression longitudinales, chacun des sommets proximaux du premier élément de stent et des sommets distaux du second élément de stent ayant une partie façonnée qui est plate ou concave et ayant une largeur, de manière à présenter une ligne de surfaces de mise en prise avec les sommets distaux du second élément de stent et les sommets proximaux du premier élément de stent respectivement, les espaces entre les surfaces de mise en prise circonférentiellement adjacentes étant plus étroits que les largeurs des parties façonnées lorsque la prothèse est dans une configuration comprimée ;
chacun de la pluralité d'éléments de stent étant espacé d'un élément de stent adjacent par un espacement (d) de l'ordre de zéro à 20 % d'une longueur longitudinale des éléments de stent dans une configuration comprimée de la prothèse ;
les éléments de stent adjacents étant dans un agencement décalé dans lequel les sommets proximaux d'un élément de stent sont latéralement décalés des sommets distaux de l'élément de stent adjacent.

2. Prothèse selon la revendication 1, les parties façonnées étant sensiblement planes.

3. Prothèse selon la revendication 2, les parties façonnées étant sensiblement perpendiculaires à la direction longitudinale de la prothèse (110 ; 210).

4. Prothèse selon la revendication 2 ou 3, les sommets proximaux (122 ; 226a) du premier élément de stent (116a ; 216a) étant sensiblement parallèles aux sommets distaux (124 ; 224b) du second élément de stent (116b ; 216b).

5. Prothèse selon l'une quelconque des revendications précédentes, les éléments d'entretoise (120) étant sensiblement parallèles lorsque la prothèse est dans une configuration comprimée.

6. Prothèse selon l'une quelconque des revendications précédentes, chaque élément de stent (116, 116a, 116b ; 216a, 216b ; 316a) étant espacé d'un élément de stent adjacent par au moins un raccord (118).

7. Prothèse selon la revendication 6, comprenant en outre au moins un élément de mise en prise (220 ; 320) situé à une extrémité de la prothèse, l'élément de mise en prise comprenant une extension latérale (222 ; 322) qui s'étend de manière adjacente aux sommets distaux (224a ; 324a) du premier élément de stent (216a ; 316a) de sorte que l'extension latérale vient en prise avec les sommets distaux du premier élément de stent lorsque la prothèse est soumise à des forces de compression longitudinales, l'extension latérale étant façonnée pour former une surface de mise en prise sensiblement plane faisant face aux sommets distaux du premier élément de stent.

8. Prothèse selon l'une quelconque des revendications précédentes, les éléments de stent (116, 116a, 116b ; 216a, 216b ; 316a) étant fixés à un matériau de greffe pour former une endoprothèse.

9. Prothèse selon la revendication 8, les éléments de stent (116, 116a, 116b ; 216a, 216b ; 316a) étant fixés au greffon de telle sorte que chaque élément de stent est espacé d'un élément de stent adjacent.

10. Procédé de fabrication d'une prothèse sensiblement tubulaire (110 ; 210), le procédé comprenant les étapes de :
formation d'une pluralité d'éléments de stent (116, 116a, 116b ; 216a, 216b ; 316a) positionnés longitudinalement le long de la prothèse, chaque élément de stent comprenant une pluralité d'éléments d'entretoise (120) reliés par des sommets (122, 124 ; 224a, 224b, 226a ; 324a) ;
agencement de la prothèse de telle sorte que les sommets proximaux (122 ; 226a) d'un premier élément de stent (116a ; 216a) viennent en prise avec les sommets distaux (124 ; 224b) d'un second élément de stent (116b ; 216b) longitudinalement adjacent lorsque la prothèse est soumise à des forces de compression longitudinales ; et
formation de chacun des sommets proximaux du premier élément de stent et des sommets distaux du second élément de stent pour avoir une partie façonnée qui est plate ou concave et pour avoir une largeur, de manière à présenter une ligne de surfaces de mise en prise avec les sommets distaux du second élément de stent et les sommets proximaux du premier élément de stent respectivement, les espaces entre les surfaces de mise en prise circonférentiellement adjacentes étant plus étroits que les largeurs des parties façonnées lorsque la prothèse est dans une configuration comprimée ;
chacun de la pluralité d'éléments de stent étant espacé d'un élément de stent adjacent par un espacement (d) de l'ordre de zéro à 20 % d'une longueur longitudinale des éléments de stent dans une configuration comprimée de la prothèse ;
les éléments de stent adjacents étant dans un agencement décalé dans lequel les sommets proximaux d'un élément de stent sont latéralement décalés des sommets distaux de l'élément de stent adjacent.

11. Procédé selon la revendication 10, les parties façonnées étant formées pour être sensiblement planes.

12. Procédé selon la revendication 11, les parties façonnées étant formées pour être sensiblement perpendiculaires à la direction longitudinale de la prothèse (110 ; 210).

13. Procédé selon la revendication 11 ou 12, les sommets proximaux (122 ; 226a) du premier élément de stent (116a ; 216a) étant formés pour être sensiblement parallèles aux sommets distaux (124 ; 224b) du second élément de stent (116b ; 216b).

14. Procédé selon l'une quelconque des revendications 10 à 13, les éléments de stent (116, 116a, 116b ; 216a, 216b ; 316a) étant formés de telle sorte que les éléments d'entretoise (120) sont sensiblement parallèles lorsque la prothèse (110 ; 210) est dans une configuration comprimée.

15. Procédé selon l'une quelconque des revendications 10 à 14, la prothèse (110 ; 210) étant formée de telle sorte que chaque élément de stent (116, 116a, 116b ; 216a, 216b ; 316a) est espacé d'un élément de stent adjacent par au moins un raccord (118).
